# EUROPÄISCHE PATENTSCHRIFT

(11) **EP 2 755 610 B1**
(45) Veröffentlichungstag und Bekanntmachung des Hinweises auf die Patenterteilung: **06.12.2017**
(21) Anmeldenummer: 12766931.5
(22) Anmeldetag: 11.09.2012
(51) Int. Cl.: A61F 5/01

(54) **UNTERSCHENKELORTHESE**
LOWER LEG ORTHOSIS
ORTHESE DE JAMBE

(30) Priorität: 14.09.2011 DE 102011082700
(43) Veröffentlichungstag der Anmeldung: 23.07.2014
(73) Patentinhaber: Deutsches Zentrum für Luft- und Raumfahrt e.V., 51147 Köln (DE)
(72) Erfinder: RITTWEGER, Jörn, 53604 Bad Honnef (DE); DUCOS, Michel, 28199 Bremen (DE); WEBER, Tobias, 50668 Köln (DE); BRÜGGEMANN, Gert-Peter, 50858 Köln (DE)
(74) Vertreter: dompatent von Kreisler Selting Werner - Partnerschaft von Patent- und Rechtsanwälten mbB
(86) Internationale Anmeldenummer: PCT/EP2012/067730
(87) Internationale Veröffentlichungsnummer: WO 2013/037769

(56) Entgegenhaltungen:
- GB-A- 2 400 562
- US-A- 5 464 385
- US-A1- 2010 198 360
- US-B1- 6 514 293
- US-B1- 7 294 114
- US-B1- 8 142 381

## Beschreibung

Die vorliegende Erfindung betrifft eine Unterschenkelorthese mit einer Schiene zur Ruhigstellung der Unterschenkelmuskulatur, wobei die Schiene aus einer Unterschenkelmanschette und einer Fußauflage besteht.

Unterschenkelorthesen werden üblicherweise bei Verletzungen des Sehnen- und Bandapparates im Sprunggelenk eingesetzt. Sie dienen zur Ruhigstellung des Unterschenkels, wodurch weiteren Schädigungen vorgebeugt und eine Heilung ermöglicht werden soll. Die bekannten Unterschenkelorthesen basieren auf dem Prinzip einer Bewegungseinschränkung. Bekannte Unterschenkelorthesen bestehen dabei aus einer gepolsterten Schale, in die der Fuß und Unterschenkel eines Patienten gelegt und über Verschlussbänder an dem Unterschenkel und Fuß fixiert wird. Die Schale liegt dabei im Bereich der Wade und unter dem Fuß an. Entsprechende Orthesen sind beispielsweise unter der Bezeichnung "Malleo Immobil Walker" der Firma Otto Bock bekannt.

Das Grundprinzip derartiger Unterschenkelorthesen liegt in der mechanischen Bewegungseinschränkung der Plantarflexion. Während des Ganges erfolgt ein Abrollen des Fußes über eine gerundete Sohle, die an der Schale der Orthese angebracht ist. Eine derartige Orthese, die in der Sohle ein Stoßdämpferelement in Form einer Federvorrichtung aufweist, ist aus US 5,464,385 A bekannt.

Es hat sich jedoch herausgestellt, dass das Gangbild eines Patienten durch das Tragen einer derartigen Unterschenkelorthese gestört ist, da die beim normalen Gang ablaufenden mechanischen Vorgänge, nämlich die Erzeugung von Drehmomenten beim Abrollen des Fußes sowie eine zyklische Speicherung und Freisetzung von elastischer Energie, gestört sind.

Darüber hinaus führt die mechanische Bewegungseinschränkung von bekannten Orthesen nicht automatisch zu einer Ruhigstellung der Unterschenkelmuskulatur. Die Unterschenkelorthese lässt weiterhin Muskelkontraktionen zu. Ein Heilungserfolg, der eine Ruhigstellung der Muskulatur voraussetzt, ist somit zu einem großen Teil von der Mitarbeit des Patienten abhängig.

Durch die Störung des Gangbildes beim Tragen einer herkömmlichen Unterschenkelorthese wird ferner die Gehgeschwindigkeit des Patienten verringert, was zu einem gesteigerten Zeit- und Energiebedarf führt. Dadurch wird das Tragen einer herkömmlichen Unterschenkelorthese als unangenehm empfunden.

Es ist daher die Aufgabe der vorliegenden Erfindung, eine Unterschenkelorthese bereitzustellen, die eine verbesserte Ruhigstellung der Muskulatur im Unterschenkel ermöglicht und darüber hinaus das Gangbild des Patienten verbessert. Die erfindungsgemäße Unterschenkelorthese ist durch den Patentanspruch 1 definiert.

Die Erfindung sieht eine Unterschenkelorthese mit einer Schiene zur Ruhigstellung der Unterschenkelmuskulatur vor, wobei die Schiene aus einer Unterschenkelmanschette und einer mit der Unterschenkelmanschette verbundenen Fußauflage besteht. Die Erfindung ist durch eine Fußvorrichtung gekennzeichnet, die unterhalb der Fußauflage angeordnet ist. Über die Fußvorrichtung stützt sich die Fußauflage während des Gehens bodenseitig ab. Die Fußvorrichtung weist eine Bodenauflagefläche und einen elastischen Energiespeicher auf. Der elastische Energiespeicher ist zwischen der Fußauflage und der Bodenauflagefläche angeordnet. Über die Fußvorrichtung sind ferner Drehmomente von der Fußauflage auf die Bodenauflagefläche übertragbar.

Durch die unter der Fußauflage angeordnete Fußvorrichtung mit einem elastischen Energiespeicher wird ein gegenüber den bekannten Unterschenkelorthesen harmonischeres Gangbild erreicht, da über den elastischen Energiespeicher eine zyklische Speicherung und Freisetzung von Energie erfolgen kann, was zu dem harmonischeren Gangbild führt. Da sich die Fußauflage nicht direkt auf dem Boden abstützt, sondern ein Abstützen über die Fußvorrichtung erfolgt, wird dem Vorfuß des Patienten der Boden als Widerlager entzogen, so dass eine Kraftentfaltung der Muskulatur an dem Unterschenkel verringert bzw. unterbunden werden kann.

Die erfindungsgemäße Unterschenkelorthese beruht somit auf einem gegenüber den herkömmlichen Orthesen unterschiedlichen Grundprinzip. Während die herkömmliche Orthese versucht, die Bewegung des Fußes zu verhindern, wird mit der erfindungsgemäßen Orthese die Kraftentfaltung der Muskulatur im Unterschenkel verhindert. Der Vorfuß des Patienten ist somit entlastet und muss nicht wie bei herkömmlichen Orthesen fixiert werden. Es ist daher auch nicht notwendig, dass ein Widerstand gegen die Plantarflexion des Fußes durch die erfindungsgemäße Orthese bereitgestellt wird.

Darüber hinaus kann durch den elastischen Energiespeicher während des Gehens Energie zwischengespeichert werden, so dass ein harmonisches Gangbild mit geringerem Energieverbrauch entsteht.

Bei herkömmlichen Orthesen wird ein Drehmoment als Bodenreaktionskraft von der Wadenmuskulatur erzeugt und über den Vorfuß in den Boden eingeleitet. Die erfindungsgemäße Orthese hingegen ermöglicht eine Erzeugung von Drehmomenten, z.B. über das Körpergewicht, wobei das Drehmoment durch eine Krafteinleitung über das Schienbein in die Unterschenkelmanschette erfolgen kann und von der über die Fußauflage über die Fußvorrichtung auf die Bodenauflagefläche zum Einleiten der Bodenreaktionskraft übertragen wird.

Die Erfindung sieht in vorteilhafter Weise vor, dass ein elastischer Energiespeicher über einen Flansch mit der Fußauflage verbunden ist. Dabei ist der Flansch vorzugsweise konzentrisch zu dem Unterschenkel des Patienten angeordnet. Dies hat den Vorteil, dass die Gewichtskraft des Körpers des Patienten in gerader Linie in die Fußvorrichtung über den Unterschenkel eingeleitet wird. Der Flansch ermöglicht ferner ein Lösen der Fußvorrichtung von der Fußablage, so dass die Fußvorrichtung ausgetauscht werden kann.

Der elastische Energiespeicher kann eine erste Fußplatte aufweisen, die die Bodenauflagefläche mit dem Flansch verbindet. Dabei kann vorgesehen sein, dass die erste Fußplatte elastisch ausgebildet ist. Es hat sich herausgestellt, dass eine derartige elastische erste Fußplatte in vorteilhafter Weise als elastischer Energiespeicher dienen kann. Dabei ist insbesondere vorgesehen, dass die erste Fußplatte eine Blattfeder ist.

Zusätzlich oder alternativ kann vorgesehen sein, dass der elastische Energiespeicher ein elastisches Gelenk aufweist, über das die erste Fußplatte mit dem Flansch verbunden ist. Eine Energiespeicherung während des Gehens kann somit in dem elastischen Gelenk erfolgen.

Die Speicherung von Energie in dem elastischen Gelenk und/oder der elastisch ausgebildeten ersten Fußplatte ermöglicht in vorteilhafter Weise ein zyklisches Energiespeichern während des Gehens, indem durch die Gewichtskraft des Körpers des Patienten eine Kraft auf den elastischen Energiespeicher ausgeübt wird und somit eine potentielle Energie in dem elastischen Energiespeicher gespeichert werden kann. Nach dem Abrollen der Fußvorrichtung über die Bodenauflagefläche findet eine Entlastung des elastischen Energiespeichers statt, so dass die potentielle Energie freigegeben wird und beim Abstoßen der Fußvorrichtung von dem Boden zurückgeführt wird, wodurch ein harmonischer Gang entsteht. Dadurch wird ein energiesparendes Gehen mit der erfindungsgemäßen Orthese ermöglicht.

Die elastisch ausgebildete Fußplatte kann auch starr mit dem Flansch verbunden sein, so dass die Energiespeicherung ausschließlich in der ersten Fußplatte erfolgt.

In einem Ausführungsbeispiel der Erfindung ist vorgesehen, dass die Fußvorrichtung eine zweite Fußplatte aufweist, die mit dem elastischen Energiespeicher verbunden ist, wobei die zweite Fußplatte die Bodenauflagefläche aufweist. Durch das Vorsehen einer zweiten Fußplatte kann die Bodenauflagefläche wesentlich vergrößert werden und es können beispielsweise Antirutscheigenschaften verwirklicht werden. Die zweite Fußplatte kann beispielsweise als Schuhsohle ausgebildet sein. Insbesondere kann vorgesehen sein, dass die erste Fußplatte in einem spitzen Winkel zu der zweiten Fußplatte verläuft.

Die Erfindung sieht in vorteilhafter Weise vor, dass die Unterschenkelmanschette eine Schale aufweist, die im Gebrauch an dem Schienbein anliegt. Dabei kann die Schale an den Unterschenkel des jeweiligen Patienten angepasst sein. Über die Schale der Unterschenkelmanschette lässt sich in vorteilhafter Weise die durch das Körpergewicht bereitgestellte Kraft in die Unterschenkelprothese einleiten, wodurch die auf die Bodenauflagefläche zu übertragenden Drehmomente erzeugt werden.

Die Fußauflage kann eine Fersenanlage und eine Mittelfußauflage aufweisen.

Die Schale und die Fußauflage können einteilig ausgebildet sein, wodurch eine besonders gute Anpassung der Unterschenkelorthese an den Unterschenkel und den Fuß des Patienten erfolgen kann.

Es kann vorgesehen sein, dass die Schale und/oder die Fußauflage aus einem Glasfaserwerkstoff bestehen. Dadurch ist eine sehr stabile und sehr leichte Ausbildung der erfindungsgemäßen Orthese möglich.

In einem Ausführungsbeispiel der Erfindung ist vorgesehen, dass die Unterschenkelmanschette eine Befestigungsvorrichtung aufweist, über die die Unterschenkelmanschette an dem Unterschenkel des Patienten fixierbar ist. Die Befestigungsvorrichtung kann beispielsweise aus Gurtbändern bestehen.

Im Folgenden wird die Erfindung unter Bezugnahme auf die nachfolgende Figur näher erläutert.

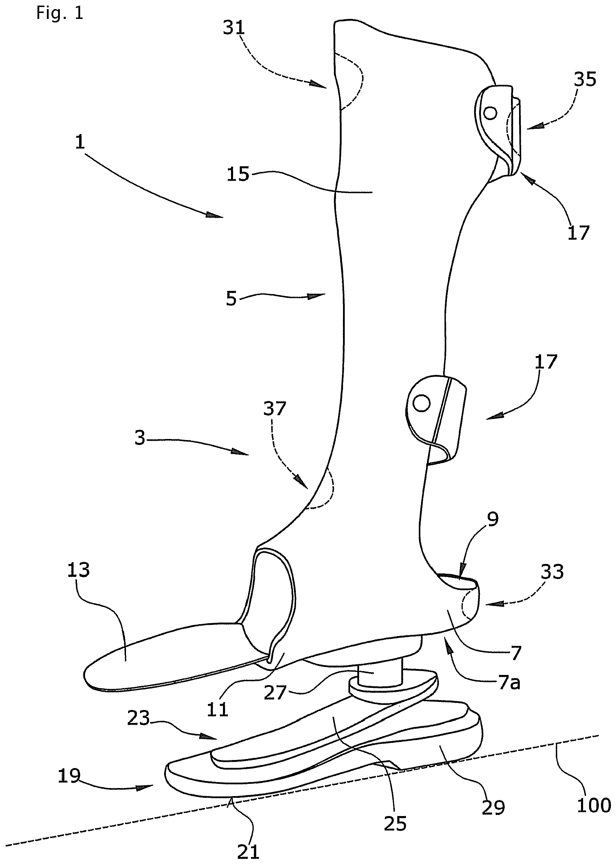

In der einzigen Figur ist eine schematische perspektivische Ansicht einer erfindungsgemäßen Unterschenkelorthese 1 gezeigt. Der Unterschenkel und Fuß des Patienten sind in der Figur nicht dargestellt. Die Unterschenkelorthese 1 weist eine Schiene 3 zur Ruhigstellung der Unterschenkelmuskulatur eines Patienten auf, wobei die Schiene 3 aus einer Unterschenkelmanschette 5 und einer mit der Unterschenkelmanschette 5 verbundenen Fußauflage 7 besteht.

Die Fußauflage 7 ist einstückig mit der Unterschenkelmanschette 5 ausgebildet. Die Fußauflage 7 besteht aus einer Fersenanlage 9 und einer Mittelfußauflage 11. Ferner kann die Fußauflage 7 eine Vorfußplatte 13 aufweisen, die eine geringe Biegesteifigkeit aufweist. Diese dient als Auflage für den Vorfuß und reduziert die Reibung und daraus resultierende Hautirritationen im Bereich der Fußsohle des Patienten.

Die Unterschenkelmanschette 5 weist eine Schale 15 auf, die an dem Unterschenkel des Patienten anliegt. Die Schale 15 ist an den Unterschenkel des Patienten angepasst. An der Unterschenkelmanschette 5 ist eine Befestigungsvorrichtung 17 angeordnet, die aus zwei an der Schale 15 befestigten Befestigungsmitteln, beispielsweise Befestigungsgurten, besteht. Über die Befestigungsvorrichtung 17 ist die Unterschenkelmanschette 5 an dem Unterschenkel des Patienten fixierbar.

An der Unterseite 7a der Fußauflage 7 ist eine Fußvorrichtung 19 angeordnet, über die sich die Fußablage 7 auf einem Boden 100 abstützen kann. Die Fußvorrichtung 19 weist eine Bodenauflagefläche 21 und einen elastischen Energiespeicher 23 auf. Der elastische Energiespeicher 23 besteht aus einer ersten Fußplatte 25, die elastisch, beispielsweise als Blattfeder, ausgebildet ist. Die erste Fußplatte 25 ist über einen Flansch 27 mit der Fußauflage 7 verbunden. Der Flansch 27 ist dabei unterhalb des Unterschenkels des Patienten, vorzugsweise konzentrisch mit dem Unterschenkel, angeordnet, so dass die Gewichtskraft über den Unterschenkel geradlinig in die Fußvorrichtung 19 eingeleitet wird. Über den Flansch 27 ist die Fußvorrichtung 19 von der Unterschenkelmanschette 5 lösbar, so dass die Fußvorrichtung 19 ausgetauscht werden kann oder mit anderen Unterschenkelmanschetten 5 verwendbar ist. Die Unterschenkelmanschette 5 ist an den Unterschenkel des Patienten angepasst, so dass die Fußvorrichtung 19 als standardisiertes Teil vorgesehen sein kann, das über den Flansch 27 mit der Unterschenkelmanschette 5 verbunden wird.

Die Fußvorrichtung 19 weist ferner eine zweite Fußplatte 29 auf, die mit der ersten Fußplatte 25 verbunden ist. Die zweite Fußplatte 29 weist in dem in der Figur dargestellten Ausführungsbeispiel die Bodenauflagefläche 21 auf und ist in Form einer Schuhsohle ausgebildet. Die erste Fußplatte 25 verläuft in einem spitzen Winkel zu der zweiten Fußplatte 29. Die zweite Fußplatte 29 kann beispielsweise Anti-Rutsch-Eigenschaften aufweisen.

Die erfindungsgemäße Unterschenkelorthese 1 ermöglicht eine verbesserte Ruhigstellung des Unterschenkels eines Patienten, da die Kraftentwicklung in der Unterschenkelmuskulatur verhindert wird. Aufgrund der geringen Biegesteifigkeit der Vorfußplatte 13 weist der Vorfuß somit kein oder nur ein geringes Widerlager auf, so dass eine Kraftentwicklung in dem Unterschenkel bei der Bewegung des Vorfußes vermieden wird.

Darüber hinaus wird beim Gehen die Gewichtskraft des Patienten in den elastischen Energiespeicher 23 eingeleitet, so dass dieser einen Teil der Gewichtskraft als potentielle Energie speichert. In dem in der Figur gezeigten Ausführungsbeispiel entsteht dies durch eine Biegung der elastischen ersten Fußplatte 25. Die Energiespeicherung erfolgt während eines Abrollvorganges der Fußvorrichtung 19, der dem Abrollen eines Fußes während einer normalen Bewegung nachempfunden wird. Dazu wird Drehmoment von der Fußauflage 7 auf die Fußvorrichtung 19 übertragen. Das Drehmoment wird durch das Körpergewicht des Patienten erzeugt und von dem Schienbein in die Unterschenkelmanschette 5 eingeleitet. Dazu muss das Schienbein zumindest an einem ersten Anlagepunkt 31, der in der Figur schematisch angedeutet ist, an der Unterschenkelmanschette 5 anliegen. Ferner ist es von Vorteil, wenn an der Fußauflage 7 an der Fersenanlage 9 ein zweiter Anlagepunkt 33 vorgesehen ist. Über zumindest diese beiden Anlagepunkte 31,33 wird das Drehmoment erzeugt und in die Fußvorrichtung 19 eingeleitet und an die Bodenauflagefläche 21 zum Erzeugen einer Bodenreaktionskraft übertragen. Dabei wird bei der Übertragung von Drehmomenten eine elastische Biegung der ersten Fußplatte 25 hervorrufen, wodurch eine Speicherung von potentieller Energie ermöglicht wird. Während des Abrollens der Fußvorrichtung über die zweite Bodenplatte 29 wird die potentielle Energie wieder freigegeben und dem System zurückgeführt, so dass ein energieeffizienter und harmonischer Gang ermöglicht wird.

Beim Aufsetzen der Fußvorrichtung 19 auf den Boden 100 stützt sich der Unterschenkel des Patienten an einem dritten Auflagepunkt 35, der im oberen Bereich der Befestigungsvorrichtung 17 angeordnet ist und einen vierten Anlagepunkt 37 im unteren Bereich des Schienbeins an der Unterschenkelmanschette 5 ab.

Die Drehmomente werden in vorteilhafter Weise von dem Unterschenkel des Patienten auf die erfindungsgemäße Unterschenkelorthese 1 übertragen, wenn der erste Anlagepunkt 31 und der zweite Anlagepunkt 33 bzw. der dritte Anlagepunkt 35 und der vierte Anlagepunkt 37 in vertikaler Richtung einen möglichst großen Abstand aufweisen. Ferner sollten die Anlagepunkte möglichst großflächig an dem Unterschenkel des Patienten anliegen, um Hautirritationen und Druckstellen zu vermeiden.

Die erfindungsgemäße Orthese 1 ist insbesondere bei der Heilung von seriellen und parallelen Strukturen des Unterschenkels, nämlich den Bändern, Sehnen, Kapseln, Muskeln oder auch bei Knochenbrüchen im Fußbereich einsetzbar. Die Orthese kann auch bei einer Frühmobilisierung nach einer Komplettruhigstellung, beispielsweise nach einer Verletzung oder Operation, eingesetzt werden, oder bei lähmungs- oder altersbedingten Gangstörungen. Die erfindungsgemäße Unterschenkelorthese 1 ermöglicht insbesondere eine Entlastung der Unterschenkelmuskulatur in ihrer Funktion während des Schrittzyklus.

In dem in der Figur dargestellten Ausführungsbeispiel ist die erste Fußplatte 25 starr mit dem Flansch 27 verbunden. Selbstverständlich ist es auch möglich, dass die erste Fußplatte 25 über ein Drehgelenk, das elastisch ausgebildet ist, beispielsweise über eine drehelastische Feder, mit dem Flansch verbunden ist. Dabei kann die erste Fußplatte 25 elastisch oder auch mit hoher Biegesteifigkeit ausgebildet sein.

Der elastische Energiespeicher kann eine veränderliche Steifigkeit aufweisen. Somit kann die erfindungsgemäße Unterschenkelorthese beispielsweise an das Gewicht eines Patienten oder an die Gang- und Laufgeschwindigkeit angepasst werden.

Die erfindungsgemäße Unterschenkelorthese ermöglicht in besonders vorteilhafter Weise einen harmonischen Gang des Patienten, da die zyklische Energiespeicherung und Energierückführung während eines Schrittzyklus ermöglicht wird. Selbstverständlich ist es notwendig, dass an dem gesunden Bein eines Patienten der durch die Fußvorrichtung 19 entstehende Höhenunterschied ausgeglichen wird, indem beispielsweise eine Verdickung der Schuhsohle vorgesehen ist.

Darüber hinaus wird die Ruhigstellung der Unterschenkelmuskulatur wesentlich verbessert, da die Kraftentfaltung in der Muskulatur nahezu vollständig unterbunden wird. Dadurch wird die Heilung wesentlich verbessert und durch den ermöglichten harmonischeren Gang wird das Tragen der erfindungsgemäßen Unterschenkelorthese für den Patienten angenehmer.

## Patentansprüche

1. Unterschenkelorthese (1) mit einer Schiene (3) zur Ruhigstellung der Unterschenkelmuskulatur,
wobei die Schiene (3) aus einer Unterschenkelmanschette (5) und einer mit der Unterschenkelmanschette (5) verbundenen Fußauflage (7) besteht,
eine Fußvorrichtung (19) unterhalb der Fußauflage (7) angeordnet ist, über die sich die Fußauflage (7) bodenseitig abstützt,
wobei die Fußvorrichtung (19) eine Bodenauflagefläche (21) und einen elastischen Energiespeicher (23) aufweist, der zwischen der Fußauflage (7) und der Bodenauflagefläche (21) angeordnet ist,
wobei der Energiespeicher (23) zur zyklischen Energiespeicherung und Energierückführung während des Gehens dient,
**dadurch gekennzeichnet , dass** über den elastischen Energiespeicher (23) der Fußvorrichtung (19) Drehmomente von der Fußauflagefläche (7) auf die Bodenauflagefläche (21) übertragbar sind.

2. Unterschenkelorthese nach Anspruch 1, **dadurch gekennzeichnet, dass** der elastische Energiespeicher (23) über einen Flansch (27) mit der Fußauflage (7) verbunden ist.

3. Unterschenkelorthese nach einem der Ansprüche 1 oder 2, **dadurch gekennzeichnet, dass** der elastische Energiespeicher (23) eine erste Fußplatte (25) aufweist, die die Bodenauflagefläche (21) mit dem Flansch (27) verbindet.

4. Unterschenkelorthese nach Anspruch 3, **dadurch gekennzeichnet, dass** die erste Fußplatte (25) elastisch ausgebildet ist.

5. Unterschenkelorthese nach Anspruch 4, **dadurch gekennzeichnet, dass** die erste Fußplatte (25) eine Blattfeder ist.

6. Unterschenkelorthese nach einem der Ansprüche 3 bis 5, **dadurch gekennzeichnet, dass** der elastische Energiespeicher (23) ein elastisches Gelenk aufweist, über das die erste Fußplatte (25) mit dem Flansch (27) verbunden ist.

7. Unterschenkelorthese nach einem der Ansprüche 3 bis 5, **dadurch gekennzeichnet, dass** die erste Fußplatte (25) starr mit dem Flansch (27) verbunden ist.

8. Unterschenkelorthese nach einem der Ansprüche 1 bis 7, **dadurch gekennzeichnet, dass** die Fußvorrichtung (19) eine zweite Fußplatte (29) aufweist, die mit dem elastischen Energiespeicher (23) verbunden ist, wobei die zweite Fußplatte (29) die Bodenauflagefläche (21) aufweist.

9. Unterschenkelorthese nach Anspruch 8, **dadurch gekennzeichnet, dass** die zweite Fußplatte (29) als Schuhsohle ausgebildet ist.

10. Unterschenkelorthese nach einem der Ansprüche 1 bis 9, **dadurch gekennzeichnet, dass** die Unterschenkelmanschette (5) eine Schale (15) aufweist, die an dem Schienbein anliegt.

11. Unterschenkelorthese nach Anspruch 10, **dadurch gekennzeichnet, dass** die Schale (15) an den Unterschenkel angepasst ist.

12. Unterschenkelorthese nach einem der Ansprüche 1 bis 11, **dadurch gekennzeichnet, dass** die Fußauflage (7) eine Fersenanlage (9) und eine Mittelfußauflage (11) aufweist.

13. Unterschenkelorthese nach einem der Ansprüche 10 bis 12, **dadurch gekennzeichnet, dass** die Schale (15) und die Fußauflage (7) einteilig ausgebildet sind.

14. Unterschenkelorthese nach einem der Ansprüche 1 bis 13, **dadurch gekennzeichnet, dass** die Schale (15) und/oder die Fußauflage (7) aus einem Glasfaserwerkstoff bestehen.

15. Unterschenkelorthese nach einem der Ansprüche 1 bis 14, **dadurch gekennzeichnet, dass** die Unterschenkelmanschette (5) eine Befestigungsvorrichtung (17) aufweist, über die die Unterschenkelmanschette (5) an dem Unterschenkel fixierbar ist.

## Claims

1. A lower-leg orthosis (1) with a splint (3) for the immobilization of the muscles of the lower leg,
the splint (3) being formed by a lower-leg cuff (5) and a foot rest (7) connected with the lower-leg cuff (5),
a foot device (19) is arranged below the foot rest (7), via which the foot rest (7) is supported on the ground,
the foot device (19) comprising a ground contact surface (21) and an elastic energy reservoir (23) arranged between the foot rest (7) and the ground contact surface (21),
wherein the energy reservoir (23) serves for a cyclic energy storage and energy feedback during walking,
**characterized in that**
via the elastic energy reservoir (23) of the foot device (19), torques can be transferred from the foot rest (7) to the ground contact surface (21).

2. The lower-leg orthosis of claim 1, **characterized in that** the elastic energy reservoir (23) is connected with the foot rest (7) via a flange (27).

3. The lower-leg orthosis of one of claims 1 or 2, **characterized in that** the elastic energy reservoir (23) comprises a first foot plate (25) connecting the ground contact surface (21) with the flange (27).

4. The lower-leg orthosis of claim 3, **characterized in that** the first foot plate (25) is elastic.

5. The lower-leg orthosis of claim 4, **characterized in that** the first foot plate (25) is a leaf spring.

6. The lower-leg orthosis of one of claims 3 to 5, **characterized in that** the elastic energy reservoir (23) comprises an elastic joint via which the first foot plate (25) is connected with the flange (27).

7. The lower-leg orthosis of one of claims 3 to 5, **characterized in that** the first foot plate (25) is rigidly connected with the flange (27).

8. The lower-leg orthosis of one of claims 1 to 7, **characterized in that** the foot device (19) comprises a second foot plate (29) connected with the elastic energy reservoir (23), the second foot plate (29) comprising the ground contact surface (21).

9. The lower-leg orthosis of claim 8, **characterized in that** the second foot plate (29) is designed as a shoe dole.

10. The lower-leg orthosis of one of claims 1 to 9, **characterized in that** the lower-leg cuff (5) comprises a shell (15) resting on the shinbone.

11. The lower-leg orthosis of claim 10, **characterized in that** the shell (15) is adapted to the lower leg.

12. The lower-leg orthosis of one of claims 1 to 11, **characterized in that** the foot rest (7) comprises a heel abutment (9) and a metatarsus rest (11).

13. The lower-leg orthosis of one of claims 10 to 12, **characterized in that** the shell (15) and the foot rest (7) are formed integrally.

14. The lower-leg orthosis of one of claims 1 to 13, **characterized in that** the shell (15) and/or the foot rest (7) are made of a fiberglass material.

15. The lower-leg orthosis of one of claims 1 to 14, **characterized in that** the lower-leg cuff (5) comprises a fastening device (17) by which the lower-leg cuff (5) can be fastened on the lower leg.

## Revendications

1. Orthèse de la jambe (1) avec un rail (3) destiné à l'immobilisation de la musculature de la jambe,
le rail (3) se composant d'une jambière (5) et d'un appui pour le pied (7) raccordé à la jambière (5),
un dispositif de pied (19) étant disposé au-dessous de l'appui pour le pied (7) par le biais duquel l'appui pour le pied (7) s'appuie côté sol,
le dispositif de pied (19) présentant une surface d'appui au sol (21) et un accumulateur d'énergie (23) élastique qui est disposé entre l'appui pour le pied (7) et la surface d'appui au sol (21),
l'accumulateur d'énergie (23) servant à l'accumulation cyclique d'énergie et au renvoi d'énergie pendant la marche,
**caractérisée en ce que**
des couples peuvent être transmis à partir de l'appui pour le pied (7) vers la surface d'appui au sol (21) par le biais de l'accumulateur d'énergie (23) élastique du dispositif de pied (19).

2. Orthèse de la jambe selon la revendication 1, **caractérisée en ce que** l'accumulateur d'énergie (23) élastique est raccordé à l'appui pour le pied (7) par le biais d'une bride (27).

3. Orthèse de la jambe selon l'une des revendications 1 ou 2, **caractérisée en ce que** l'accumulateur d'énergie (23) élastique présente une première plaque de pied (25) qui raccorde la surface d'appui au sol (21) à la bride (27).

4. Orthèse de la jambe selon la revendication 3, **caractérisée en ce que** la première plaque de pied (25) est constituée de façon élastique.

5. Orthèse de la jambe selon la revendication 4, **caractérisée en ce que** la première plaque de pied (25) est un ressort à lame.

6. Orthèse de la jambe selon l'une des revendications 3 à 5, **caractérisée en ce que** l'accumulateur d'énergie (23) élastique présente une articulation élastique par le biais de laquelle la première plaque de pied (25) est raccordée à la bride (27).

7. Orthèse de la jambe selon l'une des revendications 3 à 5, **caractérisée en ce que** la première plaque de pied (25) est raccordée de façon rigide à la bride (27).

8. Orthèse de la jambe selon l'une des revendications 1 à 7, **caractérisée en ce que** le dispositif de pied (19) présente une deuxième plaque de pied (29) qui est raccordée à l'accumulateur d'énergie (23) élastique, la deuxième plaque de pied (29) présentant la surface d'appui au sol (21).

9. Orthèse de la jambe selon la revendication 8, **caractérisée en ce que** la deuxième plaque de pied (29) est constituée en tant que semelle de chaussure.

10. Orthèse de la jambe selon l'une des revendications 1 à 9, **caractérisée en ce que** la jambière (5) présente une coque (15) qui est adjacente au tibia.

11. Orthèse de la jambe selon la revendication 10, **caractérisée en ce que** la coque (15) est adaptée à la jambe.

12. Orthèse de la jambe selon l'une des revendications 1 à 11, **caractérisée en ce que** l'appui pour le pied (7) présente un système de talon (9) et un appui de pied central (11).

13. Orthèse de la jambe selon l'une des revendications 10 à 12, **caractérisée en ce que** la coque (15) et l'appui pour le pied (7) sont constitués d'une seule pièce.

14. Orthèse de la jambe selon l'une des revendications 1 à 13, **caractérisée en ce que** la coque (15) et/ou l'appui pour le pied (7) se composent d'un matériau en fibre de verre.

15. Orthèse de la jambe selon l'une des revendications 1 à 14, **caractérisée en ce que** la jambière (5) présente un dispositif de fixation (17) par le biais duquel la jambière (5) peut être fixée sur la jambe.
